(19) Europäisches Patentamt
European Patent Office
Office européen des brevets

(11) EP 2 081 533 B1

(12) **EUROPEAN PATENT SPECIFICATION**

(45) Date of publication and mention of the grant of the patent:
**19.10.2011 Bulletin 2011/42**

(21) Application number: **06829074.1**

(22) Date of filing: **17.11.2006**

(51) Int Cl.:
***A61F 13/15*** *(2006.01)*

(86) International application number:
**PCT/EP2006/011068**

(87) International publication number:
**WO 2008/058565 (22.05.2008 Gazette 2008/21)**

(54) **ABSORBENT ARTICLES COMPRISING A PEROXY COMPOUND AND AN ORGANIC ZINC SALT**

SAUGFÄHIGE ARTIKEL MIT EINER PEROXY-VERBINDUNG UND EINEM ORGANISCHEN ZINKSALZ

ARTICLES ABSORBANTS COMPRENANT UN COMPOSÉ PEROXY ET UN SEL DE ZINC ORGANIQUE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**29.07.2009 Bulletin 2009/31**

(73) Proprietor: **SCA Hygiene Products AB**
**405 03 Göteborg (SE)**

(72) Inventor: **WÄSTLUND-KARLSSON, Jan**
**431 49 Mölndal (SE)**

(74) Representative: **HOFFMANN EITLE**
**Patent- und Rechtsanwälte**
**Arabellastraße 4**
**81925 München (DE)**

(56) References cited:
**EP-A- 1 034 804**
**WO-A-2005/081811**
**DE-A1- 19 937 871**
**GB-A- 1 282 889**
**GB-A- 1 477 571**
**GB-A- 2 084 872**
**US-A1- 2003 135 172**
**US-A1- 2005 005 869**
**US-A1- 2006 036 222**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 081 533 B1

## Description

The present invention relates to an absorbent article such as a diaper, panty diaper, sanitary napkin or incontinence device comprising an effective odour control system. The present invention relates in particular to such absorbent articles wherein a peroxy compound and an organic zinc salt such a zinc ricinoleate interact to reduce malodours such as ammonia.

TECHNICAL BACKGROUND

One important area of development in the area of absorbent articles of the above-mentioned type is the control of odourous compounds forming typically after the release of body fluids, especially over a longer period of time. These compounds include fatty acids, ammonia, amines, sulphur-containing compounds and ketones and aldehydes. They are present as natural ingredients of body fluids or result from degradation processes of natural ingredients such as urea, which is broken down by microorganisms or bacteria occurring in the urogenital flora to ammonia.

Various approaches exist to suppress the formation of unpleasant odours in absorbent articles. WO 97/46188, WO 97/46190, WO 97/46192, WO 97/46193, WO 97/46195 and WO 97/46196 teach for instance the incorporation of odour inhibiting additives or deodorants such as zeolites and silica. The absorption of bodily liquids reduces however the odour inhibiting capacity of zeolites as soon as these become saturated with water, as mentioned for instance in WO 98/17239.

A second approach involves the addition of lactobacilli with the intention of inhibiting malodour-forming bacteria in the product. The incorporation of lactobacilli and their favourable effect are disclosed for instance in SE 9703669-3, SE 9502588-8, WO 92/13577, SE 9801951-6 and SE 9804390-4.

Another approach is the use of partially neutralized superabsorbent materials (acidic superabsorbent materials) (see WO 98/57677, WO 00/35503 and WO 00/35505).

Therefore, an ongoing demand exists in the art for effective odour-control systems in absorbent articles. In particular, it would be desirable to provide an odour-control system which achieves efficient odour reduction while maintaining the bacterial flora in the urogenital region.

US 2006/0036223 and US 2006/0036222 relate to absorbent articles comprising an absorbent body, a bodyside liner, a bodily exudate modifying agent, and a skin care formulation. The skin care formulation comprises a film forming agent and optionally a bodily exudate modifying agent neutralizer. The bodily exudate modifying agent is described as being capable of reducing the viscosity of bodily exudates which facilitates absorption of the exudate into the absorbent article and away from the skin. The above U.S. references disclose certain pairs of bodily exudate modifying agent and bodily exudate modifying agent neutralizer. For instance, the bodily exudate modifying agent may be an enzyme. In this case, the corresponding neutralizer may be an enzyme inhibitor. A large number of enzyme inhibitors is exemplified in the references, amongst those zinc salts of both saturated and unsaturated monocarboxylic acids. When the bodily exudate modifying agent is a reducing agent, the corresponding neutralizer may be an oxidizing agent. The oxidizing agent may be selected from citric acid, malic acid, alphahydroxy acid, hydrogen peroxide and peroxide. As will be appreciated from the above discussion, the references fail to disclose an absorbent article comprising a peroxy compound and an organic zinc salt in combination.

US 2003/0135172 describes absorbent articles comprising a storage layer for aqueous fluids. Odor control means are also mentioned. Concretely, zeolites, silica, carbon, chelants, antimicrobial agents, perfuming ingredients, masking agents, and mixtures thereof are given as examples of odor control materials. Many antimicrobial agents are exemplified. Preferred antimicrobials are stated as including quaternary ammonium, phenolic, amide, acid and nitro compounds, and mixtures thereof. The lengthy list of preferred acids includes zinc undecylenate. As further compounds for odor control there are mentioned low pH-derivatives, peroxides, hydrogen carbonates, extracts of vegetables, etherical oils, boron derivatives, poly-alpha-aminoacids, imides, polyimides, pvp-iodine, chitosan, polyglycoside, and cyclophanes. As such, US 2003/0135172 fails to disclose the concurrent presence of peroxy compound and organic zinc salt.

WO 2005/081811 relates to compositions that are described as being useful for maintaining the clean impression of a textile product, especially textile floor covering products. The aqueous compositions comprise at least one antimicrobial compound, at least one non-discoloring enzyme inhibitor and at least one odor-reacting compound that reacts with amine and thiol, wherein the odor-reacting compound is substantially odor-free. The odor-reacting compound may be an oxidizing agent selected from the group consisting of hydrogen peroxide; non-transitional metal salts of perborate, percarbonate, persulfate, peroxyacetic acid; m-chloroperoxybenzoic acid; dibenzoyl peroxide; chloramines; bromamines; chlorine oxide; and hypochloride compounds. In addition, the aqueous compositions of WO 2005/081811 may comprise an odor-absorbing compound selected from the group consisting of activated carbon, zeolites, zinc oxide, cyclodextrin and zinc ricinoleate.

EP 1 034 804 A1 is concerned with absorbent articles like sanitary napkins and pantiliners which comprise an oxidizing agent having a reduction potential higher than the reduction potential of the reaction $Fe^{2+}$ to $Fe^{3+}$, together

with a hemolytic agent. Thereby, the oxidizing agent may be selected from a broad range of compounds, including hydrogen peroxide.

GB 1 282 889 A pertains to deodorants for cosmetic and commercial use. These deodorants comprise at least one calcium, aluminium, magnesium or zinc salt of an unsaturated aliphatic hydroxycarboxylic acid having at least 17 carbon atoms. As an example of such salts, zinc ricinoleate is mentioned.

From other technical areas it is further known that organic zinc salts of unsaturated hydroxylated fatty acids such as zinc ricinoleate are deodorizing active ingredients (see for instance DE 1792074 A1, DE 2548344 A1 and DE 3808114 A1).

As will be appreciated from the above, the prior art is not aware of the favorable, in particular synergistic effects of using a peroxy compound in combination with an organic zinc salt.

It is one technical object of the present invention to overcome deficiencies discussed above in connection with the prior art.

It is one further technical object to provide an absorbent article having an efficient odour control system.

It is one further technical object of the present invention to considerably reduce or eliminate ammonia formation in absorbent articles.

Further objects will become apparent from the following description of the invention.

## BRIEF DESCRIPTION OF THE INVENTION

The present invention relates to an absorbent article, such as a diaper, panty diaper, panty liner, sanitary napkin or incontinence device comprising a liquid-permeable topsheet, a (preferably liquid-impermeable) backsheet and an absorbent core enclosed between said liquid-permeable topsheet and said backsheet, wherein said absorbent core comprises a peroxy compound and an organic zinc salt.

The present inventors have found that peroxy compounds and organic zinc salt, such as zinc ricinoleate interact favorably in the suppression of unpleasant odours. Accordingly, the peroxy compound and organic zinc salt have a different chemical structure and cooperate to achieve this effect.

Without wishing to be bound by theory, the mechanism underlying the odour reduction of the present invention is assumed to be as follows. It was found that the ammonia which produces the malodour in absorbent products, such as incontinence products is formed in the following way:

$$\text{Bacteria} + \text{Urea} \rightarrow NH_3$$

In the present invention, the peroxy compound, e.g. the hydrogen peroxide, has the function of suppressing bacterial growth while the organic zinc salt, e.g. the zinc ricinoleate removes the ammonia ($NH_3$) actually formed.

The aim of the present invention is to develop an absorbent article where the amount of unwanted bacteria, such as ammonia-producing bacteria does not increase during use.

## DETAILED DESCRIPTION OF THE INVENTION

Throughout the specification and claims, the use of "comprising" is intended to cover also the more restricting meanings "essentially consisting of" and "consisting".

As "absorbent article" we understand articles capable of absorbing body fluids such as urine, watery feces, female secretion or menstrual fluids. These absorbent articles include, but are not limited to diapers, panty diapers, panty liners, sanitary napkins or incontinence device (as used for instance for adults).

Such absorbent articles have a liquid-pervious topsheet, which during use is facing the wearer’s body. They further comprise a (preferably liquid-impervious) backsheet, for instance a plastic film, a plastic-coated nonwoven or a hydrophobic nonwoven and an absorbent core enclosed between the liquid-pervious topsheet and the backsheet.

A suitable topsheet may be manufactured from a wide range of materials such as woven and nonwoven materials (e.g. a nonwoven web of fibers), polymeric materials such as apertured plastic films, e.g. apertured formed thermoplastic films and hydroformed thermoplastic films; porous foam; reticulated foams; reticulated thermoplastic films; and thermoplastic scrims. Suitable woven and nonwoven materials can be comprised of natural fibers (e.g. wood or cotton fibers), synthetic fibers (e.g. polymeric fibers such as polyesters, polypropylene or polyethylene fibers) or from a combination of natural and synthetic fibers. When the topsheet comprises a nonwoven web, the web may be manufactured by a wide number of known techniques. For example, the web may be spun-bonded, carded, wet-laid, meltblown, hydroentangled, combinations of the above or the like. In accordance with the invention, it is preferred to make use of apertured plastic films (e.g. thermoplastic films) or nonwoven materials based on synthetic fibers, e.g. those made from

polyethylene or polypropylene homo- or copolymers and polymer compositions based thereon.

Optionally, at least one further layer exists between the absorbent core and the topsheet and may be made from hydrophobic and hydrophilic web or foam materials. As "web material" we understand coherent flat fiber-based structures of paper tissue, woven or nonwoven type. The nonwoven material may have the same features as described above for topsheets.

Specifically, the at least one further layer may contribute to fluid management, for instance in the form of at least one acquisition/ distribution layer. Such structures are taught for instance by US 5,558,655, EP 0 640 330 A1, EP 0 631 768 A1 or WO 95/01147.

"Foam materials" are also well known in the art and for instance describe in EP 0 878 481 A1 or EP 1 217 978 A1 in the name of the present applicant.

The absorbent core may be partially or totally surrounded by a core wrap. It comprises an absorbent material that is generally compressible, conformable, non-irritating to the wearer's skin and capable of absorbing and retaining liquids such as urine and other body exudates.

Examples for absorbent materials include a wide variety of liquid-absorbent materials commonly used in disposable diapers and other absorbent articles such as comminuted wood pulp, which is generally referred to as air felt or fluff, as well as creped cellulose wadding; melt blown polymers, including co-form; chemically stiffened, modified or crosslinked cellulosic fibers; tissue, including tissue wraps and tissue laminates, absorbent foams, absorbent sponges, superabsorbent polymers (such as superabsorbent fibers or particles), absorbent gelling materials, or any other known absorbent materials or combinations of materials.

The fibers typically present in the absorbent core are preferably also capable of absorbing body liquid as is the case for hydrophilic fibers. Most preferably the fibers are cellulosic fibers such as wood pulp fluff, cotton, cotton linters, rayon, cellulose acetate and the like, the use of cellulosic fluff pulp being preferred. The cellulosic fluff pulp can be of mechanical or chemical type, the chemical pulp being preferred.

The absorbent core preferably comprises superabsorbent polymers and/or cellulosic fluff pulp fibers. If used in admixture, the SAP/fluff pulp weight ratio is preferably 20/80 to 70/30, e.g. 30/70 to 60/40.

The term "superabsorbent polymers" is well known in the art and designates water-swellable, water-insoluble materials capable of absorbing the multiple of their own weight in body fluids. Preferably, the superabsorbent polymer (SAP) is capable of absorbing at least about 10 times its weight, preferably at least about 15 times its weight, in particular at least about 20 times its weight in an aqueous solution containing 0.9 wt.-% of sodium chloride (under usual measuring conditions where the superabsorbent surface is freely accessible to the liquid to be absorbed). To determine the absorption capacity of the superabsorbent polymer, the standard test EDANA WSP 241.2 can be used.

The superabsorbent polymer may be in any form suitable for use in absorbent articles including particles, fibers, flakes, spheres and the like, the particle form being preferred.

According to one embodiment, the superabsorbent polymer in the absorbent core comprises an acidic superabsorbent since acidic components may exert a beneficial influence on odour control. In an alternative embodiment, the absorbent core in the absorbent article does not contain an acidic superabsorbent material, in particular an acidic superabsorbent material having a pH of s 5.5. Thereby, the pH of both standard (i.e. non-acidic) and acidic SAP is measured using the standard test EDANA WSP 200.2.

SAPs are based on homo- or copolymers comprising at least one polymerizable unit having an acidic group (e.g. a carboxylic acid group or a sulfonic acid group) such as methacrylic acid, acrylic acid, maleic acid, vinylsulfonic acid. The corresponding polymers include, but are not limited to poly(meth)acrylic acids, ethylene maleic anhydride copolymers, polymers and copolymers of vinylsulfonic acids, polyacrylates, acrylic acid grafted starch and isobutylene maleic anhydride copolymers. These polymers are preferably crosslinked to render the materials substantially water insoluble. According to one preferred embodiment of the present invention, the superabsorbent material is a crosslinked homo- or copolymer comprising (meth)acrylic acid units, for instance of the type disclosed in EP 0 391 108 A2. Standard SAPs have a pH which lies e.g. in a range of 5.8 or more.

There are two ways of manufacturing acidic SAP. One way is to add an acid, e.g. citric acid, to a standard SAP, thereby reducing the pH. The other method is to maintain a low degree of neutralisation. A standard SAP has a high percentage (typically at least 70%) of the acidic groups neutralised under formation of alkali metal salts. In contrast thereto, acidic SAPs manufactured according to this method have a lower degree of neutralisation, typically 15 to 60%. The degree of neutralisation and pH strongly correlate which implies that the acidity of the SAP can be controlled by the degree of neutralisation.

As used herein, "peroxy compound" means a compound comprising at least one peroxy group (-O-O-) in the molecule. Specifically, it is a collective term covering inorganic peroxides, such as compounds of the general formula $M_2O_2$ (M being an alkali metal) or $M'O_2$ (M' being an alkaline earth metal), hydroperoxides, organic peroxides, diacyl peroxides, peracids, peracid esters, ketone peroxides, and hydrogen peroxide. These types will be further illustrated below.

Hydroperoxides are represented by the general formula R-O-OH. In the formula, R represents an organic

residue, preferably an alkyl, aryl, alkylaryl or arylalkyl group. Most preferably, R represents a linear, branched or cyclic C1-6 alkyl, C3-6 aryl or C4-C14 alkylaryl or C4-C14 arylalkyl group. The above definition of R is also applicable to the further formulae below. When there is more than one group R in the respective formula, the groups R may be selected independently from the above groups. The organic peroxides are represented by the general formula R-O-O-R. One specific example is di-tert-butyl peroxide (R = $C(CH_3)_3$). Another example is dicumyl peroxide (R = $C(CH_3)_2C_6H_5$). The peracids can be represented by the following general formula:

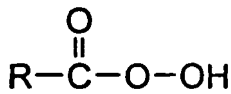

Examples of useful peracids are peracetic acid, persuccinic acid, m-chlorperbenzoic acid, peroxy benzoic acid and performic acid. Diacyl peroxides have the following general formula:

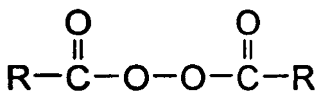

A representative example of a diacyl peroxide is dibenzoyl peroxide (*i.e.* R=$C_6H_5$). Peracid esters can be represented by the following general formula:

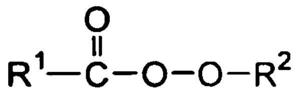

wherein $R^1$ and $R^2$ independently have the same meaning as R above. Further types of peroxides are ketone peroxides of the general formula

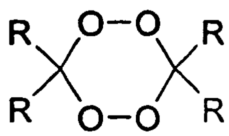

When selecting the peroxy compound for use in the present invention, proper consideration should be given to the instability of many peroxy compounds. It should also be duly considered that many peroxy compounds are irritating to skin. This may render desirable specific measures to avoid a rewet or wetting through of the absorbent product whereby the wearer's skin may come in contact with peroxy compounds dissolved in bodily liquids.

Taking account of the above, hydrogen peroxide is preferably used in the present invention. It has the additional advantage of being commercially available as a 3 and 30% solution. According to the Merck Index, the 3% hydrogen peroxide solution contains 2.5 to 3.5 wt.-% $H_2O_2$, and the 30% hydrogen peroxide solution contains 30 wt.-% $H_2O_2$. The latter is commercially available from Merck as Perhydrol®. For the purpose of the present invention, the 3% hydrogen peroxide solution, which is alto sold as a topical anti-infective and antiseptic, as well as a cleansing agent is most preferred.

There are no specific restrictions regarding the organic zinc salt to be used in combination with the peroxy compound. Preferably, use of aliphatic or aromatic monocarboxylic acids is made. In accordance with the present invention, zinc salts of organic carboxylic acids having 2 to 30 carbon atoms, in particular 12 to 24 carbon atoms are preferably used. The carboxylic acid group may be attached to aliphatic, aliphatic-aromatic, aromatic-aliphatic, alicyclic, or aromatic residues, wherein the aliphatic chain or the alicyclic ring(s) may be unsaturated and are optionally substituted, for instance by hydroxy or C1 to C4 alkyl. These salts include zinc acetate, zinc lactate, zinc ricinoleate and zinc abietate. More preferably, the zinc salt is the zinc salt of an unsaturated hydroxylated fatty acid having 8 to 18 carbon atoms. Although there is no specific restriction regarding the number of unsaturated double bonds or hydroxy groups, those fatty acids having one or two unsaturated double bonds and one or two hydroxyl groups seem to be preferred. The most preferred embodiment is zinc ricinoleate. The organic zinc salt may also be activated by the presence of amino acids as in TEGO® Sorb available from Degussa. Further, the organic zinc salt to be used in the present invention may also

be capable of removing malodorous substances chemically based on amines, *e.g.*, nicotine in cigarette smoke, thiocompounds, *e.g.*, allicin in garlic and onions, and acids, *e.g.*, isovaleric acid in human sweat, and butyric acid. For instance, zinc ricinoleate which is, *e.g.*, marketed by Degussa under the tradename TEGO® Sorb has the described additional odor removing effect apart from removing ammonia.

According to a particular preferred embodiment of the present invention, the peroxy compound is hydrogen peroxide and the organic zinc salt is zinc ricinoleate.

As regards the amount of peroxy compound and organic zinc salt to be used in the present invention, there are no specific restrictions. In the present specification, these amounts are expressed in relation to the weight (in g) of the dry absorbent core.

Herein the term "dry" used in relation to the absorbent core is to be understood such that no water has been added to the absorbent core and that the only water present in the absorbent core is the unavoidable residual water from manufacturing. For the purpose of the present invention, an absorbent core is preferably regarded as "dry" after a circular test core having a thickness of 5 to 6 mm, a diameter of 5 cm and which has been compressed to a bulk of about 8-10 $cm^3/g$ has been kept for at least one week at ambient temperature (e.g. 20 °C) and a specified relative humidity, for example 50% RH.

While there are no specific restrictions as to the amount of peroxy compound to be used in the present invention, as long as the object of the present invention is not compromised, the amount of peroxy compound is preferably at least $1 \times 10^{-5}$ g, more preferably at least $1 \times 10^{-4}$ g, still more preferably at least $5 \times 10^{-4}$ g, most preferably at least $1 \times 10^{-3}$ g peroxy compound per g of dry absorbent core. Beyond a certain amount of peroxy compound (for instance 0.01 g or 0.1 g per g dry absorbent core), it may no longer be economical to add further peroxy compound. Moreover, using too high an amount of peroxy compound may be disadvantageous in view of the irritating effect of many peroxy compounds on skin. When the absorbent core comprises pulp, peroxy compound and organic zinc salt, and optionally SAP, it is preferred to use the peroxy compound in an amount of 0.01 to 0.1 wt.-% based on pulp.

Very low amounts of organic zinc salts cooperate already with antibacterials or alkali metal chloride in a very efficient odour control. A preferred lower weight limit of organic zinc salt (calculated as zinc), such as zinc ricinoleate seems to be at least $1 \times 10^{-5}$ g per g of dry absorbent core. More preferred values are at least $1 \times 10^{-4}$ g, even more preferably at least $5 \times 10^{-4}$ g per g dry absorbent core, even more preferably at least $1 \times 10^{-3}$ g per g of dry absorbent core. There is no specific upper limit, even though for economic reasons, a point may be reached where it may no longer be useful to further increase the zinc content, for instance to values of 0.1 or 1 g zinc per g absorbent core, if this is not accompanied by an enhanced odour suppression.

The weight ratio of the organic zinc salt to the peroxy compound is also not specifically limited, but it is preferably 15/1 to 1/5, more preferably 5/1 to 1/2, for instance 3/1 to 1/1.

The present invention is also not subject to any limitations regarding the technique of incorporating the peroxy compound and the organic zinc salt into the absorbent core. However, dipping and spraying are preferred.

For instance, it is conceivable to treat the pulp fibers to be used in the absorbent core, preferably cellulosic fluff pulp with a mixed solution of the peroxy compound and the organic zinc salt prior to or during admixture with the SAP. In the alternative, the fibers to be used in the absorbent core can be treated successively with separate solutions, e.g. by dipping and spraying, a first solution comprising the peroxy compound, and a second solution comprising the organic zinc salt.

According to one alternative embodiment, the superabsorber (SAP) to be used in the absorbent core is treated with a mixed solution of the peroxy compound and the organic zinc salt prior to or during admixture with the pulp fibers, in particular cellulosic fluff pulp. In the alternative, the SAP to be used in the absorbent core can be treated successively with separate solutions, e.g. by dipping and spraying, a first solution comprising the peroxy compound, and a second solution comprising the organic zinc salt.

According to a preferred embodiment, a mixed solution of the organic zinc salt and the peroxy compound is sprayed onto the fibers, most preferably onto the cellulosic fluff pulp sheets as obtained from the manufacturer. The mixed solution can be sprayed onto the fluff pulp sheet directly by the manufacturer of these sheets prior to the delivery of the sheets to the manufacturer of the absorbent articles.. This is an especially preferred embodiment since it avoids the extra step of spraying the mixed solution or the separate solutions (i.e. of the organic zinc salt and the antibacterial or chloride) when manufacturing the absorbent article. Alternatively, the pulp sheet is dipped into the solution. The SAP can be added during or after sheet formation to obtain an absorbent core treated in accordance with the present invention.

The incorporation of peroxy compound and organic zinc salt can be achieved by treating an already formed absorbent core comprising a mixture of pulp fibers, preferably cellulosic fluff pulp and optionally SAP with a solution containing the organic zinc salt, in particular the zinc ricinoleate solution as well as the peroxy compound.

According to one preferred application technique, the solution(s) of the peroxy compound and the organic zinc salt, in particular zinc ricinoleate is/are sprayed on one or both sides of the absorbent core, or one of both sides of the individual layers constituting the same.

The most preferred way is to pre-treat the SAP and/or the pulp fibers, such as the fluff pulp fibers, by adding

a mixed solution or separate solutions of the organic zinc salt and the peroxy compound, and to incorporate these components into the absorbent core during core formation.

The solvent used for the mixed solution of peroxy compound and organic zinc salt can be water, a preferably volatile organic solvent such as ethanol or a mixture of water and a water-miscible organic solvent such as ethanol, as long as the peroxy compound used will dissolve therein. Preferably, these solvents are also used when preparing two separate solutions of the peroxy compound and the organic zinc salt. In the case of the two solutions, the solvents can be selected independently dependent on the solubility of the peroxy compound and the organic zinc salt. Preferably, the peroxy compound and organic zinc salt are present in the solution(s) in a relatively high concentration, preferably 1 to 30 wt.-%. The use of such concentrated solutions ensures that the absorption capacity of the superabsorbent material is not impaired more than necessary. As solutions of peroxy compounds, in particular peroxides having a relatively high concentration tend to be less stable than more diluted solutions, one of average skill in the art will readily select suitable concentrations of peroxy compound in consideration of the conflicting aims of using solutions having a concentration which is sufficiently high to avoid unnecessary impairment of the absorption capacity of the SAP while insuring sufficient stability of the solution. Commercially available solutions of organic zinc salts such as TEGO® Sorb A30 available from Degussa (content of actives 30 weight%, zinc ricinoleate activated by an amino acid), optionally in a diluted form, to which an appropriate amount of desired peroxy compound (e.g. aqueous solution of the peroxy compound) is added, can also be employed.

The backsheet typically prevents the exudates absorbed by the absorbent layer and contained within the article from soiling other external articles that may contact the absorbent article, such as bed sheets and undergarments. In preferred embodiments, the backsheet is substantially impervious to liquids (e.g., urine) and comprises a laminate of a nonwoven and a thin plastic film such as a thermoplastic film having a thickness of about 0.012 mm to about 0.051 mm. Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the absorbent article while still preventing exudates from passing through the backsheet. Exemplary breathable materials may include materials such as woven webs, nonwoven webs, composite materials such as film-coated nonwoven webs, and microporous films. Since there is always a trade-off between breathability and liquid-impermeability it can be desired to provide backsheets showing a certain, relatively minor liquid-permeability but very high breathability values.

The above elements of an absorbent article can be assembled, optionally together with other typical elements of absorbent articles in a manner known in the art.

The present invention is also concerned with cellulosic fibers, in particular cellulosic fluff pulp fibers comprising a peroxy compound and an organic zinc salt as specified above. The cellulosic fibers can be obtained by treating them in a manner as described above.

The following examples and comparative examples illustrate the present invention without limiting same.

EXAMPLE

Circular test absorbent cores having a weight of about 1.16 g and a diameter of 5 cm were punched out of an absorbent core produced in a pilot plant. A standard method of mat forming a core was used in the production of the core in the pilot plant. The absorbent core consisted of a homogenous mixture of fluff pulp and superabsorbent material. The fluff pulp used was 0.69 g Weyerhauser pulp (NB 416) and the superabsorbent material was 0.47 g of a superabsorber (SXM 9155, Degussa). The absorbent core was compressed to a bulk of about 8-10 $cm^3/g$.

To the absorbent core 1.3 ml of a 0.5 wt.-% solution of zinc ricinoleate (available from Degussa under the tradename TEGO® Sorb A30 and suitably diluted), corresponding to an amount of $5.36 \times 10^{-4}$ g Zn per g dry absorbent core and hydrogen peroxide in an amount of 0.075 wt.-% in terms of the fluff pulp in the absorbent core, corresponding to an amount of $4.50 \times 10^{-4}$ g hydrogen peroxide per g dry absorbent core were added by either dripping the solution onto the surface (on one side) or dipping one side of the core into the solution. One week after the treatment, the absorbent body was allowed to absorb 16 ml synthetic urine according to Method A as described below and then allowed to stand at room temperature.

Method A: Measurement of ammonia inhibition in absorbent cores

Absorbent cores were prepared as described above. Test liquid 1 was prepared. Bacteria suspension of Proteus mirabilis was cultivated in nutrient broth 30°C overnight. The graft cultures were diluted and the bacterial count was determined. The final culture contained approximately $10^5$ organisms per ml of test liquid. The absorbent core was placed in a plastic jar and the Test liquid 1 was added to the absorbent core, whereafter the container was incubated at 35°C for 6 hours, whereafter samples were taken from the containers using a hand pump and a so called Drdger-tube. The ammonia content was obtained as a colour change on a scale graded in ppm or volume percent.

Test liquid 1:

Sterile synthetic urine to which has been added a growth medium for micro-organisms. The synthetic urine contains mono- and divalent cations and anions and urea and has been prepared in accordance with the information in Geigy, Scientific Tables, Vol 2, 8th ed. 1981 p. 53. The growth medium for the micro-organisms is based on information of Hook- and FSA-media for entero-bacteria. The pH in this mixture is 6.6.

COMPARATIVE EXAMPLE

An absorbent core was formed in the same manner as in Example 1, except that no zinc ricinoleate was used and hydrogen peroxide was used in an amount of 0.1 wt.-% in terms of the fluff pulp in the absorbent core, corresponding to an amount of 6.00 x $10^{-4}$ g hydrogen peroxide per g dry absorbent core.

6h after the absorption of synthetic urine the amount of ammonia developed was measured. Five measurements were averaged as mean value.

The results in terms of ammonia formation of the Example and Comparative Example are shown in the following Table 1.

TABLE 1

| | sample description | Ammonia Formation (ppm) 6 h |
|---|---|---|
| Comp. Example | $H_2O_2$ | 1100 |
| Example | $Zn^1 + H_2O_2$ | 47 |
| [1] Zinc ricinoleate | | |

As shown by the above data, using zinc ricinoleate in combination with a peroxy compound, such as hydrogen peroxide will allow the formation of ammonia to be largely suppressed even when the amount of hydrogen peroxide is reduced from 6.00 x $10^{-4}$ (in the Comparative Example) to 4.50 x $10^{-4}$ (in the Example) g/g absorbent core. Consequently, the above experiment shows that the combined use of a peroxy compound and an organic zinc salt such as zinc ricinoleate suppresses the formation of ammonia to a very surprising extent.

**Claims**

1. Absorbent article, such as a diaper, panty diaper, sanitary napkin or incontinence device comprising a liquid-permeable topsheet, a backsheet and an absorbent core enclosed between said liquid-permeable topsheet and said backsheet, wherein said absorbent core comprises a peroxy compound and an organic zinc salt.

2. Absorbent article according to Claim 1, wherein the absorbent core comprises a mixture of cellulosic fibers, in particular cellulosic fluff pulp, and superabsorbent material.

3. Absorbent article according to Claim 1 or 2 , wherein the peroxy compound is hydrogen peroxide.

4. Absorbent article according to any of Claims 1 to 3 , wherein the amount of peroxy compound, in particular hydrogen peroxide is at least 1 x $10^{-3}$ g per g dry absorbent core.

5. Absorbent article according to any of Claims 1 to 4, wherein the amount of organic zinc salt is such that at least 5 x $10^{-4}$ g Zn per g dry absorbent core is contained in the absorbent core .

6. Absorbent article according to any of Claims 1 to 5, wherein the weight ratio of the organic zinc salt, in terms of zinc, to the peroxy compound is 15/1 to 1/5.

7. Absorbent article according to any of Claims 1 to 6, being obtainable by treating the absorbent core with a solution of the peroxy compound and the organic zinc salt.

8. Absorbent article according to any of the preceding claims, wherein the organic zinc salt is selected from zinc salts of carboxylic acids having 2 to 30 carbon atoms.

9. Absorbent article according to Claim 8 wherein the carboxylic acid represents an unsaturated hydroxylated fatty acid having 8 to 18 carbon atoms.

10. Absorbent article according to Claim 8 or 9 wherein the organic zinc salt is zinc ricinoleate.

11. Absorbent article according to any of the preceding claims wherein the backsheet is liquid-impermeable.

12. Cellulosic fibers, in particular cellulosic fluff pulp fibers, **characterized in that** they comprise a peroxy compound and an organic zinc salt.

13. Cellulosic fibers according to Claim 12, wherein the organic zinc salt is zinc ricinoleate.

14. Cellulosic fibers according to Claim 12 or 13, wherein the peroxy compound is hydrogen peroxide.

15. Cellulosic fibers according to any of Claims 12 to 14 obtainable by treating the cellulosic fibers with the peroxy compound and the organic zinc salt.

16. A method of manufacturing cellulosic fibers according to any of Claims 12 to 15, wherein the cellulosic fibers are dipped into, or sprayed with, a first solution of the peroxy compound and a second solution of the organic zinc salt, or a mixed solution of the peroxy compound and the organic zinc salt.

## Patentansprüche

1. Absorbierender Artikel, wie beispielsweise eine Windel, Slipeinlage, Damenbinde oder Inkontinenzvorrichtung, der eine flüssigkeitsdurchlässige obere Lage, eine hintere Lage und einen absorbierenden Kern umfasst, der zwischen der flüssigkeitsdurchlässigen oberen Lage und der hinteren Lage eingeschlossen ist, worin der absorbierende Kern eine Peroxyverbindung und ein organisches Zinksalz umfasst.

2. Absorbierender Artikel gemäß Anspruch 1, worin der absorbierende Kern eine Mischung aus Cellulosefasern, insbesondere Flaumzellstoff, und ein superabsorbierendes Material umfasst.

3. Absorbierender Artikel gemäß Anspruch 1 oder 2, worin die Peroxyverbindung Wasserstoffperoxid ist.

4. Absorbierender Artikel gemäß mindestens einem der Ansprüche 1 bis 3, worin die Menge der Peroxyverbindung, insbesondere Wasserstoffperoxid, mindestens $1 \times 10^{-3}$ g pro Gramm trockenem absorbierenden Kern ist.

5. Absorbierender Artikel gemäß mindestens einem der Ansprüche 1 bis 4, worin die Menge des organischen Zinksalzes so ist, dass mindestens $5 \times 10^{-4}$ g Zn pro Gramm trockenem absorbierenden Kern im absorbierenden Kern enthalten sind.

6. Absorbierender Artikel gemäß mindestens einem der Ansprüche 1 bis 5, worin das Gewichtsverhältnis des organischen Zinksalzes, bezogen auf Zink, zur Peroxyverbindung 15/1 bis 1/5 ist.

7. Absorbierender Artikel gemäß mindestens einem der Ansprüche 1 bis 6, der durch Behandeln des absorbierenden Kerns mit einer Lösung der Peroxyverbindung und des organischen Zinksalzes erhältlich ist.

8. Absorbierender Artikel gemäß mindestens einem der vorhergehenden Ansprüche, worin das organische Zinksalz aus Zinksalzen von Carbonsäuren mit 2 bis 30 Kohlenstoffatomen ausgewählt ist.

9. Absorbierender Artikel gemäß Anspruch 8, worin die Carbonsäure eine ungesättigte hydroxylierte Fettsäure mit 8 bis 18 Kohlenstoffatomen darstellt.

10. Absorbierender Artikel gemäß Anspruch 8 oder 9, worin das organische Zinksalz Zinkricinoleat ist.

11. Absorbierender Artikel gemäß mindestens einem vorhergehenden Anspruch, worin die hintere Lage flüssigkeitsundurchlässig ist.

12. Cellulosefasern, insbesondere Flaumzellstofffasern,
**dadurch gekennzeichnet, dass** sie eine Peroxyverbindung und ein organisches Zinksalz umfassen.

13. Cellulosefasern gemäß Anspruch 12, worin das organische Zinksalz Zinkricinoleat ist.

14. Cellulosefasern gemäß Anspruch 12 oder 13, worin die Peroxyverbindung Wasserstoffperoxid ist.

15. Cellulosefasern gemäß mindestens einem der Ansprüche gemäß 12 bis 14, die durch Behandeln von Cellulosefasern mit der Proxyverbindung und dem organischen Zinksalz erhältlich sind.

16. Verfahren zum Herstellen von Cellulosefasern gemäß mindestens einem der Ansprüche 12 bis 15, worin die Cellulosefasern in eine erste Lösung der Peroxyverbindung und eine zweite Lösung des organischen Zinksalzes oder in eine gemischte Lösung der Peroxyverbindung und des organischen Zinksalzes getaucht werden oder mit diesen/ dieser besprüht werden.

**Revendications**

1. Article absorbant tel qu'une couche, une couche-culotte, une serviette hygiénique, ou un dispositif de protection contre l'incontinence, comprenant une feuille supérieure perméable aux liquides, une feuille arrière et un noyau absorbant enfermé entre ladite feuille supérieure perméable aux liquides et ladite feuille arrière, ledit noyau absorbant renfermant un composé du type peroxyde et un sel de zinc organique.

2. Article absorbant selon la revendication 1 dans lequel le noyau absorbant comprend un mélange de fibres de cellulose, en particulier de la pâte de fluff de cellulose, et de matériau superabsorbant.

3. Article absorbant selon la revendication 1 ou 2 dans lequel le composé du type peroxyde est un peroxyde hydrogéné.

4. Article absorbant selon l'une quelconque des revendications 1 à 3 dans lequel la quantité de composé du type peroxyde, en particulier de peroxyde hydrogéné est d'au moins 1 x $10^{-3}$ g par g de noyau absorbant sec.

5. Article absorbant selon l'une quelconque des revendications 1 à 4 dans lequel la quantité de sel de zinc organique est telle que le noyau absorbant contient au moins 5 x $10^{-4}$ g de Zn par g de noyau absorbant sec.

6. Article absorbant selon l'une quelconque des revendications 1 à 5 dans lequel le rapport en poids du sel de zinc organique, mesuré en zinc, au composé du type peroxyde est de 15/1 à 1/5.

7. Article absorbant selon l'une quelconque des revendications 1 à 6 pouvant être obtenu en traitant le noyau absorbant avec une solution du composé du type peroxyde et du sel de zinc organique.

8. Article absorbant selon l'une quelconque des revendications qui précèdent dans lequel le sel de zinc organique est choisi parmi les sels de zinc d'acides carboxyliques comportant de 2 à 30 atomes de carbone.

9. Article absorbant selon la revendication 8 dans lequel l'acide carboxylique consiste en un acide gras non saturé hydroxylé comportant de 8 à 18 atomes de carbone.

10. Article absorbant selon la revendication 8 ou 9 dans lequel le sel de zinc organique est le ricinoléate de zinc.

11. Article absorbant selon l'une quelconque des revendications qui précèdent dans lequel la feuille arrière est imperméable aux liquides.

12. Fibres de cellulose, en particulier des fibres de pâte de fluff de cellulose, **caractérisées en ce qu'**elles comprennent un composé du type peroxyde et un sel de zinc organique.

13. Fibres de cellulose selon la revendication 12 dans lesquelles le sel de zinc organique est le ricinoléate de zinc.

14. Fibres de cellulose selon la revendication 12 ou 13 dans lesquelles le composé du type peroxyde est un peroxyde hydrogéné.

**15.** Fibres de cellulose selon l'une quelconque des revendications 12 à 14 pouvant être obtenues en traitant les fibres de cellulose avec le composé du type peroxyde et avec le sel de zinc organique.

**16.** Procédé de fabrication de fibres de cellulose selon l'une quelconque des revendications 12 à 15, dans lequel les fibres de cellulose sont plongées dans une première solution du composé du type peroxyde, ou bien subissent une pulvérisation d'une première solution du composé du type peroxyde, et sont plongées dans une deuxième solution du sel de zinc organique, ou bien subissent une pulvérisation d'une deuxième solution du sel de zinc organique, ou bien encore sont plongées dans une solution mixte du composé du type peroxyde et du sel de zinc organique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9746188 A
- WO 9746190 A
- WO 9746192 A
- WO 9746193 A
- WO 9746195 A
- WO 9746196 A
- WO 9817239 A
- SE 97036693
- SE 95025888
- WO 9213577 A
- SE 98019516
- SE 98043904
- WO 9857677 A
- WO 0035503 A
- WO 0035505 A
- US 20060036223 A
- US 20060036222 A
- US 20030135172 A
- WO 2005081811 A
- EP 1034804 A1
- GB 1282889 A
- DE 1792074 A1
- DE 2548344 A1
- DE 3808114 A1
- US 5558655 A
- EP 0640330 A1
- EP 0631768 A1
- WO 9501147 A
- EP 0878481 A1
- EP 1217978 A1
- EP 0391108 A2


### Non-patent literature cited in the description


- **GEIGY.** Scientific Tables. 1981, vol. 2, 53